# EUROPÄISCHE PATENTSCHRIFT

(11) **EP 2 040 050 B1**
(45) Veröffentlichungstag und Bekanntmachung des Hinweises auf die Patenterteilung: **07.03.2012**
(21) Anmeldenummer: 07018676.2
(22) Anmeldetag: 24.09.2007
(51) Int. Cl.: G01J 3/44, G01J 3/02, G01N 21/17, A61B 5/00

(54) **Messanordnung für ein optisches Spektrometer**
Measuring assembly for an optical spectrometer
Dispositif de mesure pour un spectromètre optique

(43) Veröffentlichungstag der Anmeldung: 25.03.2009
(73) Patentinhaber: Universität Potsdam, 14469 Potsdam (DE)
(72) Erfinder: Reich, Oliver. Dr., 14471 Potsdam (DE); Löhmannsröben, Hans-Gerd, Prof. Dr., 14476 Postdam-Golm (DE)
(74) Vertreter: Bittner, Thomas L.

(56) Entgegenhaltungen:
- EP-A- 0 843 986
- US-A- 5 452 723
- US-A- 5 987 351
- US-A1- 2006 089 556
- US-B1- 7 054 002

## Beschreibung

Die Erfindung betrifft eine Messanordnung für ein optisches Spektrometer, insbesondere ein Photonendichtewellen-Spektrometer.

### Hintergrund der Erfindung

Spektrometer sind allgemein Mess- oder Analyseeinrichtungen zum spektroskopischen Untersuchen einer Probe. Optische Spektrometer nutzen Licht für die spektroskopische Untersuchung. Spektroskopische Untersuchungen mit Photonendichtewellen erlauben die getrennte Absolutbestimmung von Absorptions- und Streueigenschaften stark streuender Proben, die mit klassischen Methoden der Lichtstreuung oder der Absorptionsspektroskopie so nicht möglich sind. Unter Photonendichtewellen wird die zeit- und ortsabhängige Ausbreitung von Licht in einem stark streuenden Medium verstanden, welche durch die Anzahl der Photonen pro Volumen, also die Photonendichte charakterisiert ist.

Die Beschreibung der Photonenausbreitung lehnt sich an Untersuchungen über den Transport von Neutronen an, da sowohl Photonen als auch Neutronen ungeladen sind und sich, abgesehen von Streuprozessen, frei von äußeren Kräften bewegen. Allerdings vereinfacht sich die Beschreibung für Photonen wesentlich, da sie im Gegensatz zu Neutronen alle eine konstante und gleiche Geschwindigkeit besitzen. Ausgehend von der Strahlungstransportgleichung, in der alle Beiträge bilanziert werden, die zu einer Veränderung der Lichtintensität führen, wird meist eine Entwicklung in eine Reihe von Kugelflächenfunktionen verwendet, um eine einfache Beschreibung der Photonendichte zu erhalten. Hierbei tritt als charakteristische Größe analog zum Teilchentransport ein optischer Diffusionskoeffizient auf, der mittels Absorptions- und Streueigenschaften des Mediums beschrieben wird.

Da die Lichtausbreitung durch Absorption und Streuung charakteristisch beeinflusst wird, kann die Analyse von Photonendichtewellen zur Bestimmung dieser optischen Eigenschaften verwendet werde. Die bekannten experimentellen Techniken zur Nutzung von Photonendichtewellen können anhand der jeweils verwendeten Zeitabhängigkeit des eingestrahlten Lichtes unterschieden werden. Zum einen wird für Untersuchungen in der Zeitdomäne ein sehr kurzer Lichtimpuls in das Medium eingestrahlt, und dann wird ortsabhängig die Zeitabhängigkeit der Lichtausbreitung verfolgt. Zum anderen werden für Experimente in der Frequenzdomäne Lichtquellen verwendet, die in ihrer Intensität sinusförmig moduliert werden. Außerdem können Untersuchungen auch mit zeitlich konstanten Lichtquellen durchgeführt werden.

Die Spektroskopie auf Basis von Photonendichtewellen ist insbesondere zum Untersuchen stark streuender Medien mit relativ geringer Absorption geeignet. Einerseits gestattet hohe Streuung die Beschreibung der Lichtausbreitung als zufälligen Teilchentransport. Andererseits verringert sich die Photonendichte bei starker Absorption so sehr, dass die Detektion schwierig wird. Untersuchungen mittels Photonendichtewellen werden deshalb häufig in einem Spektralbereich von 600 bis 900 nm durchgeführt, wo viele Materialien nur eine mäßige Absorption zeigen. Streuung wird durch örtliche Variation des Brechungsindex hervorgerufen und ist dann besonders stark, wenn die Größenskala der Variation der Wellenlänge des Lichtes entspricht und der Unterschied im Brechungsindex groß ist. Daher zeigen insbesondere Dispersionen aus Materialien mit unterschiedlichem Brechungsindex, deren disperse Phase aus Teilchen mit einem Durchmesser von über 50 nm besteht, im sichtbaren Spektralbereich eine starke Streuung.

Bei einem Spektrometer zur Untersuchung einer Probe mittels Photonendichtewellen wird üblicherweise Anregungslicht, welches von einer Lichtquelle erzeugt wird, beispielsweise einem Laser, mittels einer Einkoppeleinrichtung auf die zu untersuchende Probe eingekoppelt. Dieses erfolgt beispielsweise unter Verwendung eines Lichtwellenleiters in der Einkoppeleinrichtung. Eine Auskoppeleinrichtung dient dann dazu, in der zu untersuchenden Probe aufgrund des eingestrahlten Anregungslichtes entstehendes Messlicht auszukoppeln und an eine Detektionseinrichtung abzugeben. Auch die Auskoppeleinrichtung kann unter Verwendung eines oder mehrerer Lichtwellenleiter gebildet sein, in welche das Messlicht eingekoppelt wird, um es zu der Detektionseinrichtung zu leiten. Unter Verwendung physikalischer Modellbetrachtungen kann das detektierte Messlicht ausgewertet werden, um optische Eigenschaften der zu untersuchenden Probe zu ermitteln.

Dem Einkoppeln des Anregungslichtes und dem Auskoppeln des Messlichtes muss besondere Aufmerksamkeit gewidmet werden, um tatsächlich auswertbare Messlichtsignale zu empfangen.

Aus dem Dokument EP 0 843 986 A2 ist eine Messvorrichtung zum nicht-invasiven Bestimmen der Glukosekonzentration in Blut bekannt, welche ein optisches Messverfahren nutzt. Hierbei wird infrarotes Anregungslicht mit Hilfe von Lichtleiterelementen auf die Hautoberfläche einer Person eingekoppelt. Mit Hilfe anderer Lichtleiterelemente wird erzeugtes Messlicht von der Hautoberfläche zu einem Detektor geführt. In einer Ausführungsform sind Ausgänge von Lichtleiterelementen zum Einkoppeln des Anregungslichtes und Ausgänge von Lichtleiterelementen zum Empfangen des Messlichtes in zwei parallelen Reihen zueinander angeordnet, die ihrerseits entlang einer Spirale verlaufen können.

Das Dokument US 7,054,002 B1 betrifft ein optisches Messsystem, mit dem Anregungslicht auf eine Messprobe eingekoppelt wird und Messlicht mit Hilfe von Lichtleiterelementen in der Probe erfasst wird. Endabschnitte der Lichtwellenleiter sind in der Probe angeordnet.

Das Dokument US 5,452,723 offenbart eine optische Messeinrichtung, bei der Anregungslicht über einen Lichtwellenleiter auf eine Probenoberfläche eingekoppelt wird. Ein hiervon getrennter Lichtwellenleiter dient zum Empfangen von Messlicht.

### Zusammenfassung der Erfindung

Aufgabe der Erfindung ist es, eine Messanordnung für ein Photonendichtewellen-Spektrometer mit verbesserten Lichtein-/Lichtauskoppeleigenschaften anzugeben.

Diese Aufgabe wird erfindungsgemäß durch eine Messanordnung für ein optisches Spektrometer nach dem unabhängigen Anspruch 1 gelöst. Vorteilhafte Ausgestaltungen der Erfindung sind Gegenstand von abhängigen Unteransprüchen.

Die Erfindung umfasst den Gedanken eines Photonendichtewellen-Spektrometers, mit einer Messkammer, welche mit einer zu messenden Probe beladbar ist, und einer Ein-/Auskoppeleinrichtung, die konfiguriert ist, Anregungslicht von einer Lichtquelle zu empfangen und auf die zu messende Probe in der Messkammer einzukoppeln und in der zu messende Probe infolge des eingekoppelten Anregungslichtes gebildetes Messlicht zu empfangen und an eine Detektionseinrichtung abzugeben, wobei die Ein-/Auskoppeleinrichtung eine optische Schalteinrichtung und mehrere hieran koppelnde Lichtleiterelemente mit einem jeweiligen Lichtwellenleiter aufweist, die mittels der optischen Schalteinrichtung gemäß zumindest einer wählbaren Messkonfiguration ihrer Nutzung entsprechend zum Abgeben des Anregungslichtes und zum Empfangen des Messlichtes beschaltbar sind, um der zumindest einen wählbaren Messkonfiguration entsprechend das Anregungslicht einzukoppeln und das Messlicht zu empfangen, und wobei Ausgänge der mehreren Lichtleiterelemente in Blickrichtung auf die Ausgänge einer spiralförmigen Anordnung entsprechend positioniert sind.

Es wurde überraschend gefunden, dass die spiralförmige Anordnung der Ausgänge der mehreren Lichtleiterelemente in besonderer Weise eine effiziente Einkopplung des Anregungslichtes sowie eine effiziente Auskopplung des Messlichtes unterstützt. Übersprecheffekte zwischen den mehreren Lichtleiterelementen sind minimiert. Die vorgeschlagene Anordnung der mehreren Lichtleiterelemente unterstützt darüber hinaus eine möglichst ungestörte Lichtausbreitung in der zu messenden Probe von dem oder den Lichtwellenleitern, welche das Anregungslicht einkoppeln, zu dem oder den Lichtwellenleitern, welche das Messlicht empfangen.

Auf diese Weise kann die Anzahl der verwendeten Lichtwellenleiter erhöht werden, wodurch sich ein großer Bereich zugänglicher

Je nach Messkonfiguration können die mehreren Lichtleiterelemente mit Hilfe der optischen Schalteinrichtung im Rahmen einer Messaufgabe so eingerichtet werden, dass sowohl das Anregungslicht auf die zu messende Probe eingekoppelt als auch das Messlicht ausgekoppelt werden. Beispielsweise kann jedes der mehreren Lichtleiterelemente nacheinander zum Einkoppeln des Anregungslichtes und zum Empfangen des Messlichtes verwendet werden, indem die optische Schalteinrichtung eine entsprechende optische Beschaltung der mehreren Lichtleiterelemente realisiert. Auch kann alternativ vorgesehen sein, dass eines der mehreren Lichtleiterelemente für die Dauer der Messung zum Einkoppeln des Anregungslichtes beschaltet wird, wohingegen einige oder alle der verbleibenden Lichtleiterelemente dem Empfang von Messlicht und dessen Weiterleitung zur Detektionseinrichtung dienen. Eine gewählte Beschaltung, die einer Messkonfiguration entspricht, kann für die Dauer einer durchzuführenden Messung unverändert bleiben. In einer anderen Ausgestaltung ändert sich die Messkonfiguration im Verlauf einer Messung einmal oder mehrfach. Je nach Beschaltung des individuellen Lichtleiterelementes wird durch den zugehörigen Lichtwellenleiter eine bestimmte Lichtart geleitet, nämlich Anregungslicht oder Messlicht. Den vorangehenden Ausführungen entsprechend ist ein Verfahren zum spektroskopischen Untersuchen einer Probe, insbesondere eine Untersuchung mittels Photonendichtewellen, in verschiedenen Varianten ausführbar.

Optische Schalteinrichtungen sind als solche beispielsweise in Form von Faser-Faser-Schaltern bekannt, mit denen Faserenden von Lichtwellenleitern relativ zueinander so positioniert werden können, dass eine Übertragung von Licht aus einem Faserende in ein anderes Faserende entweder stattfindet oder nicht. Solche optischen Schalteinrichtungen nutzen beispielsweise Translationsschlitten, um die Faserenden relativ zueinander zu verlagern. Die vorgeschlagene Messanordnung kann je nach Anwendungsfall unter Verwendung unterschiedlicher optischer Schalteinrichtungen realisiert werden.

Eine bevorzugte Weiterbildung der Erfindung sieht vor, dass die Ausgänge der mehreren Lichtleiterelemente relativ zu einer Bezugsebene quer zur Lichtaustrittsrichtung des Anregungslichtes aus den Ausgängen in unterschiedlichen Abständen angeordnet sind. Entlang der Ausbreitungsrichtung des Anregungslichtes sind die Ausgänge also versetzt zueinander gebildet. Hierbei kann auch vorgesehen sein, dass wenigstens zwei der Ausgänge im gleichen Abstand von der Bezugsebene angeordnet sind, wohingegen wenigstens ein anderer Ausgang relativ hierzu verschoben ist.

Bei einer zweckmäßigen Ausgestaltung der Erfindung kann vorgesehen sein, dass die Ausgänge der mehreren Lichtleiterelemente in ihrer Lage relativ zueinander wenigstens für die zumindest eine Messkonfiguration fixiert sind. Die Fixierung der Ausgänge der mehreren Lichtleiterelemente relativ zueinander kann dauerhaft festgelegt sein. Alternativ kann vorgeschen sein, dass die Ausgänge der mehreren Lichtleiterelemente relativ zueinander verlagerbar sind, beispielsweise mittels Verschieben, so dass die Relativpositionen zwischen unterschiedlichen Messungen oder sogar für verschiedene Messschritte innerhalb einer Messung neu eingestellt werden können, dann aber in fixierter Lage vorliegen. Eine Verlagerung relativ zueinander der Ausgänge der mehreren Lichtleiterelemente kann beispielsweise mittels geeigneter Translationsschlitten ausgeführt werden, die in als solchen bekannten Ausgestaltungen auch die notwendige Präzision einer solchen Relativverlagerung realisieren können.

Eine vorteilhafte Ausführungsform der Erfindung sieht vor, dass wenigstens ein Teil der Ausgänge der mehreren Lichtleiterelemente in der Messkammer angeordnet ist. Auf diese Weise ist es ermöglicht, dass der Teil der Ausgänge der mehreren Lichtleiterelemente, welcher in der Messkammer angeordnet ist, sogar in die zu untersuchende Probe eintaucht, was zusätzlich dadurch beeinflusst werden kann, dass der Füllgrad der Messkammer anwendungsabhängig eingestellt wird. Dieses kann für einige oder alle der in der Messkammer angeordneten Ausgänge je nach Messaufgabe vorgesehen sein.

Bevorzugt sieht eine Fortbildung der Erfindung vor, dass der Teil der Lichtleiterelemente, für den der Ausgang in der Messkammer angeordnet ist, fluiddicht durch einen Wandabschnitt der Messkammer hindurch geführt ist. In einer fluiddichten Wanddurchführung können die Lichtleiterelemente feststehend oder verlagerbar, beispielsweise mittels Schieben oder Ziehen, angeordnet sein. Beispielsweise ist das Lichtleiterelement als Lichtwellenleiter in einer Dichtung aus einem elastischen Material wie Gummi oder Kautschuk geführt.

Bei einer vorteilhaften Ausgestaltung der Erfindung kann vorgesehen sein, dass die Ausgänge der mehreren Lichtleiterelemente von Lichtwellenleiter-Ausgängen der Lichtwellenleiter gebildet sind. Alternativ hierzu können den Lichtwellenleiter-Ausgängen optische Elemente vorgelagert sein, die dann ihrerseits den unmittelbaren Ausgang für das Lichtleiterelement bilden, durch welchen das Anregungslicht austritt oder das Messlicht eintritt. Vorgesehen sein können in diesem Zusammenhang beispielsweise Linsen oder Linsensysteme, welche dann den unmittelbaren Ausgang bilden.

Eine Weiterbildung der Erfindung kann vorsehen, dass die Lichtwellenleiter aus Lichtwellenleitern mit einem Gradienten-Index-Profil gebildet sind. Die Verwendung von Lichtwellenleitern mit einem Gradienten-Index-Profil ermöglicht die Verringerung einer Demodulation und hierdurch eine Verbesserung der Messlichtqualität bei höheren Frequenzen der Lichtmodulation oder der Verwendung längerer Lichtwellenleiter.

Eine bevorzugte Weiterbildung der Erfindung sieht vor, dass die Lichtwellenleiter aus photonischen Kristallfasern gebildet sind. Beispielsweise können photonische Kristallfasern in einer sogenannten *hollow*-*core*-Ausführung verwendet werden.

Bei einer zweckmäßigen Ausgestaltung der Erfindung kann vorgesehen sein, dass die Lichtwellenleiter gemäß wenigstens einer der folgenden Bauarten ausgeführt sind: deuterierte Lichtwellenleiter und fluorierte Lichtwellenleiter. Die Verwendung von Lichtleitern mit einer Deuterierung und / oder einer Fluorierung ermöglicht die Verringerung einer Absorption und somit den Einsatz in einem breiteren Spektralbereich. Außerdem führt die verringerte Oberflächenenergie von fluorierten Materialien zu einer geringeren Verschmutzung des Lichtwellenleiters und einer leichteren Reinigung.

Auch können Lichtwellenleiter vorgesehen sein, die in beliebiger Kombination mehrere der vorangehend genannten Ausgestaltungen in sich vereinen.

### Beschreibung bevorzugter Ausführungsbeispiele der Erfindung

Die Erfindung wird im folgenden anhand von bevorzugten Ausführungsbeispielen unter Bezugnahme auf Figuren einer Zeichnung näher erläutert. Hierbei zeigen:
- Fig. 1: eine schematische Darstellung für eine Messapparatur unter Verwendung eines Pho- tonendichtewellen-Spektrometers,
- Fig. 2: eine spiralförmige Anordnung von Ausgängen mehrerer Lichtleiterelemente in Blickrichtung auf die Ausgänge und
- Fig. 3: eine schematische Darstellung mit einer Messkammer und mehreren Lichtleiterele- menten, deren Ausgänge in der Messkammer angeordnet sind.

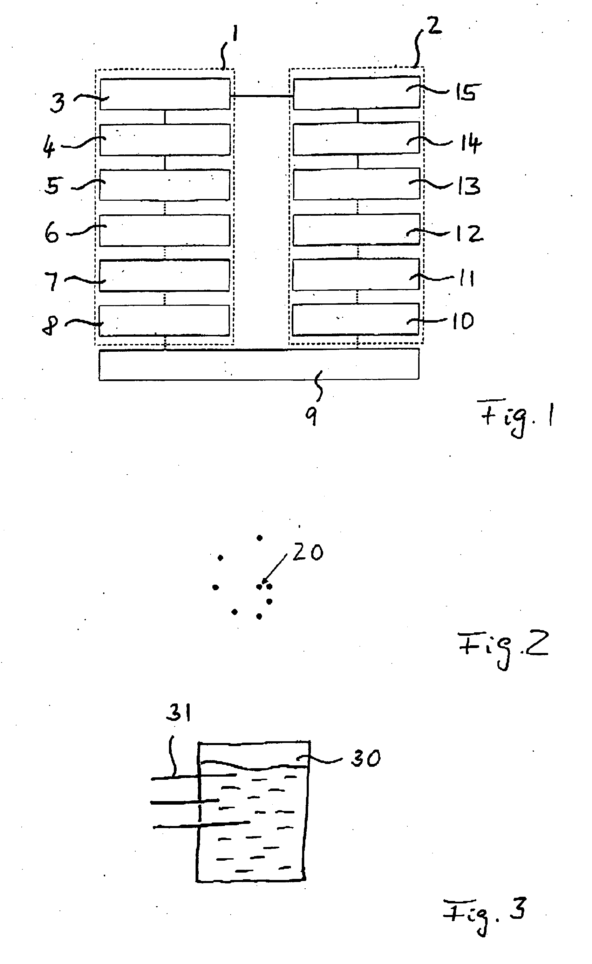

Fig. 1 zeigt eine schematische Darstellung einer Messapparatur unter Verwendung eines Photonendichtewellen-Spektrometers (PDW-Spektrometer). Hierbei beziehen sich gepunktete Linien zwischen den Kästchen auf die Übertragung optischer Signale und durchgezogenen Linien zwischen den Kästchen auf die Übertragung elektrischer Signale.

Die Messapparatur ist konzeptionell in zwei Teile gegliedert, nämlich eine Emissionseinheit 1 und eine Detektionseinheit 2. Mittels der Emissionseinheit 1 wird unter Verwendung eines Frequenzgenerators 3 ein zeitabhängiges elektrisches Signal erzeugt, welches beispielsweise einen sinusförmigen Verlauf aufweist. Der Frequenzgenerator 3 erzeugt ein moduliertes elektrisches Signal bei einer einstellbaren Frequenz. Die Frequenz liegt zwischen einigen Kilohertz und bis zu mehreren Gigahertz.

Mit Hilfe einer emissionsseitigen Signalkonditionierung 4 wird das von dem Frequenzgenerator 3 erhaltene elektrische Signal konditioniert und auf eine modulierbare Strahlungsquelle 5 gegeben, die das empfangene Signal in ein zeitabhängiges optisches Anregungslicht umwandelt. Je nach Strahlungsquelle wird das elektrische Signal zunächst konditioniert. Neben einer möglichen Verstärkung wird insbesondere über ein Biased-T ein zeitunabhängiges Signal addiert. Sollen mehrere Strahlungsquellen moduliert werden, kann ein elektronischer Hochfrequenzschalter für die gesteuerte Schaltung des Signals auf die einzelnen Strahlungsquellen vorgesehen sein.

Bei der modulierbaren Strahlungsquelle 5 handelt es sich beispielsweise um einen Laser, insbesondere ein oder mehrere Diodenlaser. Diese können direkt über den Laserstrom in ihrer Intensität moduliert werden. Weiterhin werden die Diodenlaser temperaturstabilisiert und ihre Intensität über eine Monitordiode überwacht. Die Strahlungsquelle 5 ist gegen elektromagnetische Ein- und Abstrahlung gesichert.

Das erzeugte Anregungslicht wird nach einer Konditionierung mittels einer emissionsseitigen Strahlkonditionierung 6 über emissionsseitige Strahlungstransportmittel 7 zu einer Einkoppeleinrichtung 8 geführt, von der das zeitabhängig modulierte Anregungslicht dann auf eine zu messende Probe in einer Messkammer 9 eingekoppelt wird. Der Strahlungstransport zur Probe erfolgt über Lichtwellenleiter. Hierbei wird auf eine geringe Demodulation, insbesondere bei hohen Frequenzen, sowie eine geringe Absorption bei der entsprechenden Wellenlänge geachtet. Durch den Lichtwellenleiter können erhebliche Entfernungen zwischen der Strahlungsquelle 5 und der Probe in der Messkammer 9 realisiert werden. Die Verwendung von Lichtwellenleitern mit einem Gradienten-Index-Profil oder photonischer Kristallfasern, zum Beispiel in *hollow-core-*Ausführung, erlaubt eine Verringerung der Demodulation und somit eine Verbesserung des Messsignals bei höheren Frequenzen oder eine Verwendung längerer Lichtwellenleiter. Die Verwendung von fluorierten oder / und deuterierten Lichtleitern ermöglicht die Verringerung der Absorption und somit einen breiteren spektralen Einsatzbereich. Außerdem führt die verringerte Oberflächenenergie von fluorierten Materialien zu einer geringeren Verschmutzung der Lichtwellenleiter und einer leichteren Reinigung.

In der Detektionseinheit 2 wird Messlicht, welches in der Probe aufgrund des Einkoppelns des zeitabhängigen Anregungslichtes erzeugt wird, mit Hilfe einer Auskoppeleinrichtung 10, welche ihrerseits integriert mit der Einkoppeleinrichtung 8 integriert ausgeführt ist, ausgekoppelt und über detektionsseitige Strahlungstransportmittel 11 zu einer detektionsseitigen Strahlkonditionierung 12 und anschließend dann auf einen Detektor 13 geleitet wird. Die Auskopplung aus der Probe erfolgt wiederum über einen oder mehrere Lichtwellenleiter. Diese werden über einen optischen Schalter auf einen gemeinsamen Lichtwellenleiter geschaltet oder über einen Translationsschlitten gezielt in ihrer Position in der Probe verändert werden. Der weitere Strahlungstransport von der Probe erfolgt über einen Lichtwellenleiter. Obige Erläuterungen zum Strahlungstransport des Anregungslichtes zu der Probe gelten entsprechend.

Die zu detektierende Messlichtstrahlung wird entsprechend den Anforderungen der jeweiligen Anwendung noch weiter konditioniert. So kann neben einer spektralen Filterung mittels eines (Interferenz-)filters oder auch über dichroitische Spiegel eine Aufteilung auf mehrere Detektoren erreicht werden. Dazu wird üblicherweise das optische Signal aus dem Lichtwellenleiter über eine Linse (nicht dargestellt) zunächst parallelisiert.

Das empfangene Messlicht wird von dem Detektor 13 schließlich in ein elektrisches Messsignal umgewandelt, welches seinerseits dann mit Hilfe einer detektionsseitigen Signalkonditionierung konditioniert wird, um es schließlich auf einen phasensensitiven Detektor 15 zu geben. Je nach Spektralbereich und sonstigen Bedingungen erfolgt die Umwandlung des optischen in ein elektrisches Signal über Avalanche- oder PIN-Dioden oder Photomultiplier. Dabei ist neben der zeitlichen Auflösung und spektralen Empfindlichkeit ein weiter linearer Bereich, geringes Amplituden-Phasen-Übersprechen und eine gute elektromagnetische Abschirmung erforderlich. Das elektrische Signal des Detektors 13 wird über einen Hochpass von der zeitlich unabhängigen Komponente befreit und über einen Hochfrequenzverstärker verstärkt, was mittels einer detektionsseitigen Signalkonditionierung 14 ausgeführt wird. Hierbei kann das zeitunabhängige Signal zusätzlich ausgewertet werden. Der phasensensitive Detektor 15 bestimmt die Amplitude und Phase des detektierten Signals relativ zum Signal, dass der Frequenzgenerator 3 erzeugt. Der Frequenzgenerator 3 und phasensensitiver Detektor 15 sind in einem Netzwerkanalysator kombiniert.

Einzelne Komponenten der Messapparatur in Fig. 1 können über einen Personalcomputer gesteuert werden, auf dem auch eine nähere Auswertung der Messsignale erfolgt, so dass schließlich ein optischer Absorptionskoeffizient und ein effektiver optischer Streukoeffizient ermittelt werden können. Hieraus abgeleitet können darüber hinaus andere Probenparameter ermittelt werden wie Konzentration von Teilchen oder Teilchengröße.

Fig. 2 zeigt Ausgänge mehrerer optischer Lichtleiterelemente in Blickrichtung auf die Ausgänge. Die mehreren Lichtleiterelemente werden verwendet, um einerseits das Anregungslicht auf die zu untersuchende Probe einzukoppeln und andererseits auch das in der Probe entstandene Messlicht aus dieser auszukoppeln und dann zu dem Detektor 13 abzugeben. Fig. 2 zeigt stirnseitige Enden von Lichtwellenleitern, die je nach Messaufgabe und gewählter Messkonfiguration zum Abgeben des Anregungslichtes zu der Probe oder zum Empfangen des Messlichtes aus der Probe genutzt werden können. Die der jeweiligen Messkonfiguration entsprechende Nutzung der einzelnen Lichtleiterelemente erfolgt mittels entsprechender Beschaltung, die ihrerseits mit einer optischen Schalteinrichtung (nicht dargestellt) realisiert wird, beispielsweise einem Faser-Faser-Schalter. Hierbei kann zum Beispiel ein innerer Lichtwellenleiter 20 zum Einkoppeln des Anregungslichtes genutzt werden, wohingegen verbleibende Lichtwellenleiter zum Empfang des Messlichtes dienen. Auch eine umgekehrte Ausführung kann vorgesehen sein. Weiterhin können die mehreren Lichtwellenleiter nacheinander als Lichtwellenleiter für das Anregungslicht und Lichtwellenleiter für das Messlicht verwendet werden. Der die verschiedenen Messkonfigurationen realisierende, optische Schalter kann beispielsweise unter Verwendung eines Translationsschlittens ausgeführt sein. Verschiedene Ausgestaltungen derartiger optischer Schalteinrichtungen sind als solche bekannt.

Fig. 3 zeigt eine schematische Darstellung mit einer Messkammer 30 und mehreren Lichtleiterelementen 31, deren Ausgänge in der Messkammer 30 angeordnet sind. Alternativ können die Ausgänge von oben in die Messkammer 30 hineinragen.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Photonendichtewellen-Spektrometer mit einer Messanordnung, mit:
- einer Messkammer (9; 30)), welche mit einer zu messenden Probe beladbar ist, und
- einer Ein-/Auskoppeleinrichtung (8, 10), die konfiguriert ist, Anregungslicht von einer Lichtquelle (5) zu empfangen und auf die zu messende Probe in der Messkammer (9; 30) einzukoppeln und in der zu messende Probe infolge des eingekoppelten Anregungslichtes gebildetes Messlicht zu empfangen und an eine Detektionseinrichtung (13) abzugeben,
wobei:
- die Ein-/Auskoppeleinrichtung (8, 10) eine optische Schalteinrichtung und mehrere hieran koppelnde Lichtleiterelemente mit einem jeweiligen Lichtwellenleiter aufweist, die mittels der optischen Schalteinrichtung gemäß zumindest einer wählbaren Messkonfiguration ihrer Nutzung entsprechend zum Abgeben des Anregungslichtes und zum Empfangen des Messlichtes beschaltbar sind, um der zumindest einen wählbaren Messkonfiguration entsprechend das Anregungslicht einzukoppeln und das Messlicht zu empfangen, so dass je nach Beschaltung des individuellen Lichtleiterelementes durch den zugehörigen Lichtwellenleiter eine bestimmte Lichtart, nämlich Anregungslicht oder Messlicht, geleitet wird, und
- Ausgänge der mehreren Lichtleiterelemente in Blickrichtung auf die Ausgänge einer spiralförmigen Anordnung entsprechend positioniert sind.

2. Photonendichtewellen-Spektrometer nach Anspruch 1, **dadurch gekennzeichnet, dass** die Ausgänge der mehreren Lichtleiterelemente relativ zu einer Bezugsebene quer zur Lichtaustrittsrichtung des Anregungslichtes aus den Ausgängen in unterschiedlichen Abständen angeordnet sind.

3. Photonendichtewellen-Spektrometer nach Anspruch 1 oder 2, **dadurch gekennzeichnet, dass** die Ausgänge der mehreren Lichtleiterelemente in ihrer Lage relativ zueinander wenigstens für die zumindest eine Messkonfiguration fixiert sind.

4. Photonendichtewellen-Spektrometer nach mindestens einem der vorangehenden Ansprüche, **dadurch gekennzeichnet, dass** wenigstens ein Teil der Ausgänge der mehreren Lichtleiterelemente in der Messkammer (30) angeordnet ist.

5. Photonendichtewellen-Spektrometer nach Anspruch 1, **dadurch gekennzeichnet, dass** der Teil der Lichtleiterelemente, für den der Ausgang in der Messkammer (30) angeordnet ist, fluiddicht durch einen Wandabschnitt der Messkammer (30) hindurch geführt ist.

6. Photonendichtewellen-Spektrometer nach mindestens einem der vorangehenden Ansprüche, **dadurch gekennzeichnet, dass** die Ausgänge der mehreren Lichtleiterelemente von Lichtwellenleiter-Ausgängen der Lichtwellenleiter gebildet sind.

7. Photonendichtewellen-Spektrometer nach mindestens einem der vorangehenden Ansprüche, **dadurch gekennzeichnet, dass** die Lichtwellenleiter aus Lichtwellenleitern mit einem Gradienten-Index-Profil gebildet sind.

8. Photonendichtewellen-Spektrometer nach mindestens einem der Ansprüche 1 bis 6, **dadurch gekennzeichnet, dass** die Lichtwellenleiter aus photonischen Kristallfasern gebildet sind.

9. Photonendichtewellen-Spektrometer nach mindestens einem der vorangehenden Ansprüche, **dadurch gekennzeichnet, dass** die Lichtwellenleiter gemäß wenigstens einer der folgenden Bauarten ausgerührt sind: deuterierte Lichtwellenleiter und fluorierte Lichtwellenleiter.

## Claims

1. Photon density wave spectrometer comprising a measuring arrangement, with:
- a measuring chamber (9; 30), which can be loaded with a sample to be measured, and
- a coupling-in/coupling-out device (8, 10), which is configured for receiving excitation light from a light source (5) and for coupling-in on the sample to be measured in the measuring chamber (9; 30) and for receiving measurement light formed in the sample to be measured on account of the coupled-in excitation light and for feeding it to a detection device (13),
wherein:
- the coupling-in/coupling-out device (8, 10) has an optical switching device and a plurality of optical waveguide elements coupling thereto with a respective optical waveguide, which can be connected by the optical switching device according to at least one selectable measurement configuration, to couple-in the excitation light corresponding to at least one selectable measurement configuration and to receive the measurement light, so that depending on the connection of the individual optical waveguide element a particular type of light, namely excitation light or measurement light is guided by the associated optical waveguide, and
- outputs of the several optical waveguide elements are positioned in the viewing direction towards the outputs corresponding to a spiral-shaped arrangement.

2. Photon density wave spectrometer according to claim 1, **characterized in that** the outputs of the plurality of optical waveguide elements are arranged at different distances relative to a reference level transversely to the light exit direction of the excitation light from the outputs.

3. Photon density wave spectrometer according to claim 1 or 2, **characterized in that** the outputs of the plurality of optical waveguide elements are fixed in their position relative to one another at least for the at least one measurement configuration.

4. Photon density wave spectrometer according to at least one of the preceding claims, **characterized in that** at least one part of the outputs of the plurality of optical waveguide elements is arranged in the measuring chamber (30).

5. Photon density wave spectrometer according to claim 1, **characterized in that** the part of the optical waveguide elements, for which the output is arranged in the measuring chamber (30), is led fluid-tight through a section of the wall of the measuring chamber (30).

6. Photon density wave spectrometer according to at least one of the preceding claims, **characterized in that** the outputs of the plurality of optical waveguide elements are formed of optical waveguide outputs of the optical waveguide.

7. Photon density wave spectrometer according to at least one of the preceding claims, **characterized in that** the optical waveguides are formed of optical waveguides with a graded-index profile.

8. Photon density wave spectrometer according to at least one of claims 1 to 6, **characterized in that** the optical waveguides are formed of photonic crystal fibres.

9. Photon density wave spectrometer according to at least one of the preceding claims, **characterized in that** the optical waveguides are constructed according to at least one of the following designs: deuterated optical waveguides and fluorinated optical waveguides.

## Revendications

1. Spectromètre à ondes de densité de photons comprenant un dispositif de mesure, présentant
- une chambre de mesure (9 ; 30), qui peut être chargée avec un échantillon à mesurer, et
- un dispositif d'accouplement/désaccouplement (8, 10) qui est configuré pour recevoir de la lumière d'excitation d'une source de lumière (5) et l'injecter sur l'échantillon à mesurer dans la chambre de mesure (9 ; 30) et recevoir de la lumière de mesure formée dans l'échantillon à mesurer du fait de la lumière d'excitation injectée et la céder à un dispositif de détection (13),
- le dispositif d'accouplement/désaccouplement (8, 10) présentant un dispositif de commutation optique et plusieurs éléments conducteurs de lumière se couplant à celui-ci avec un guide d'ondes lumineuses respectif, qui peuvent être commutés au moyen du dispositif de commutation optique selon au moins une configuration de mesure sélectionnable en fonction de leur utilisation pour émettre la lumière d'excitation et pour recevoir la lumière de mesure, afin d'injecter la lumière d'excitation en fonction de la au moins une configuration de mesure sélectionnable et de recevoir la lumière de mesure, et
de sorte que, après la commutation de l'élément de guide de lumière individuel par le guide d'ondes lumineuses spécifique, un type de lumière défini, à savoir de la lumière d'excitation ou de la lumière de mesure, est guidé, et
- des sorties des plusieurs éléments conducteurs de lumière sont positionnées dans le sens du regard vers les sorties en fonction d'un agencement en forme de spirale.

2. Spectromètre à ondes de densité de photons selon la revendication 1, **caractérisé en ce que** les sorties des plusieurs éléments conducteurs de lumière sont disposées à différents intervalles par rapport à un plan de référence transversalement au sens de sortie de la lumière d'excitation à partir des sorties.

3. Spectromètre à ondes de densité de photon selon la revendication 1 ou 2, **caractérisé en ce que** les sorties des plusieurs éléments conducteurs de lumière sont fixées dans leur position par rapport aux autres pour la au moins une configuration de mesure.

4. Spectromètre à ondes de densité de photons selon au moins l'une des revendications précédentes, **caractérisé en ce qu'**au moins une partie des sorties des plusieurs éléments conducteurs de lumière sont disposées dans la chambre de mesure (30).

5. Spectromètre à ondes de densité de photons selon la revendication 1, **caractérisé en ce que** la partie des éléments conducteurs de lumière, pour laquelle la sortie est disposée dans la chambre de mesure (30), est guidée de façon étanche au fluide à travers une partie de paroi de la chambre de mesure (30).

6. Spectromètre à ondes de densité de photons selon au moins l'une des revendications précédentes, **caractérisé en ce que** les sorties des plusieurs éléments conducteurs de lumière sont formées par des sorties des guides d'ondes de lumière.

7. Spectromètre à ondes de densité de photons selon au moins l'une des revendications précédentes, **caractérisé en ce que** les guides d'ondes de lumière sont constitués de guides d'ondes de lumière avec un profil d'indice de gradient.

8. Spectromètre à ondes de densité de photons selon au moins l'une des revendications 1 à 6, **caractérisé en ce que** les guides d'ondes de lumière sont formés de fibres de cristal photoniques.

9. Spectromètre à ondes de densité de photons selon au moins l'une des revendications précédentes, **caractérisé en ce que** les guides d'ondes de lumière sont réalisés selon au moins l'une des conceptions suivantes : guides d'ondes de lumière deutériés et guides d'ondes de lumière fluorés.
